# EUROPÄISCHE PATENTSCHRIFT

(11) **EP 2 785 760 B1**
(45) Veröffentlichungstag und Bekanntmachung des Hinweises auf die Patenterteilung: **21.12.2016**
(21) Anmeldenummer: 12790925.7
(22) Anmeldetag: 27.11.2012
(51) Int. Cl.: C08G 18/78, C08G 18/79, C08G 18/02, C08G 18/16

(54) **VERFAHREN ZUR HERSTELLUNG VON POLYISOCYANATEN UND DEREN VERWENDUNG**
METHOD FOR PRODUCING POLYISOCYANATES AND USE THEREOF
PROCÉDÉ DE PRODUCTION DE POLYISOCYANATES ET UTILISATION DESDITS POLYISOCYANATES

(30) Priorität: 29.11.2011 DE 102011087371
(43) Veröffentlichungstag der Anmeldung: 08.10.2014
(73) Patentinhaber: Covestro Deutschland AG, 51373 Leverkusen (DE)
(72) Erfinder: RICHTER, Frank, 51373 Leverkusen (DE); BRAHM, Martin, 51519 Odenthal (DE)
(74) Vertreter: Levpat
(86) Internationale Anmeldenummer: PCT/EP2012/073726
(87) Internationale Veröffentlichungsnummer: WO 2013/079481

(56) Entgegenhaltungen:
- EP-A1- 0 896 009
- EP-A1- 0 962 455
- EP-A1- 2 058 349

## Beschreibung

Die Oligo- bzw. Polymerisierung von Isocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und B eschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff. Eine spezielle Form der Isocyanatmodifizierung die zu Produkten mit einem hohen Anteil an Iminooxadiazindiongruppen (asymmetrischen Isocyanat-Trimeren), neben den lange bekannten Isocyanuratstrukturen (bisher vereinfachend häufig nur als "Trimere" bezeichnet) in den Verfahrensprodukten führt, wird u.a. in EP-A 962455, 962454, 896009, 798299, 447074, 379914, 339396, 315692, 295926 sowie 235388 beschrieben. Als Katalysatoren hierfür haben sich (Hydrogenpoly)fluoride bewährt, bevorzugt mit quaternären Phosphoniumkationen als Gegenion.

Ein Nachteil dieser Verfahren des Standes der Technik ist, dass sich die als Katalysator verwendeten Spezies teilweise unter Bildung störender Nebenprodukte zersetzen, was sich durch einen sukzessiv ansteigenden Phosphorgehalt des - in der Regel destillativ - wiedergewonnenen Monomers (Recyclat) äußert.

Zwar lassen sich derartig verunreinigte Recyclate aufreinigen, vgl. EP-A 1939171, jedoch ist eine derartige Vorgehensweise mit zusätzlichem Aufwand verbunden, den es zu vermeiden gilt.

Der Erfindung lag daher die Aufgabe zugrunde, ein Verfahren zur Herstellung hoch Iminooxadiazindionngruppen enthaltender Polyisocyanate zur Verfügung zu stellen, das nicht mit den vorgenannten Nachteilen behaftet ist: die Katalysatoren sollten eine bessere Stabilität im Isocyanatmilieu aufweisen und nicht bzw. im Vergleich mit Systemen des Standes der Technik weniger zur Zersetzung unter Bildung störender Nebenkomponenten neigen, die sich in den Verfahrensprodukten, insbesondere dem Recyclat, anreichern können.

Dies ist durch die Bereitstellung des erfindungsgemäßen Verfahrens gelungen.

Gegenstand der Erfindung ist die Verwendung von Katalysatoren bei der Herstellung von Iminooxadiazindiongruppen enthaltenden Polyisocyanaten durch Oligomerisierung monomerer Di- und/oder Triisocyanate, dadurch gekennzeichnet, dass der Wassergehalt der Katalysatoren bei der Zugabe zu den zu oligomerisierenden Isocyanaten 1000 ppm nicht überschreitet.

Bei Verwendung von Katalysatormischungen darf der Gesamtwassergehalt 1000 ppm nicht überschreiten.

Keiner der eingangs genannten Schriften des Standes der Technik ist zu entnehmen, dass eine Absenkung des Wassergehaltes in den zur Iminooxadiazindionbildung bevorzugten Katalysatoren des Standes der Technik zu einer signifikanten Stabilisierung dieser Spezies im Isocyanatmilieu führt. In EP 962 454 wird Wasser sogar explizit als mögliches Additiv zur Herstellung von Fluorid-Ionen enthaltenden Katalysatoren aufgeführt, die für die Herstellung von Iminooxadiazindiongruppen enthaltenden Polyisocyanaten eingesetzt werden können. Da darüber hinaus Diisocyanate selbst gegenüber Wasser reaktiv sind, war vielmehr zu erwarten, dass die 'Entwässerung' des Katalysators nach Kontakt mit dem zu trimerisierenden Isocyanat rasch einsetzen und daher eine vorgelagerte Entwässerung des Katalysators keinen Einfluß haben sollte.

Die Art und Weise, wie dem Katalysator das herstellungsbedingt enthaltene Restwasser entzogen wird (destillativ, extraktiv, durch chemische Reaktion mit einem im Verfahren unschädlichen Additiv, Adsorption etc.), ist im erfindungsgemäßen Verfahren belanglos.

Mit dem erfindungsgemäßen Modifizierverfahren ist daher eine verbesserte Methode zur Herstellung Iminooxadiazindiongruppen enthaltender Polyisocyanate in einfacher Weise zugänglich geworden.

Bevorzugte Katalysatoren sind solche auf Basis von quarternären Phophoniumsalzen, deren Kationen der allgemeinen Formel R₄P⁺ entsprechen, wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können.

Es können einzelne Phosphoniumsalze, ebenso wie Mischungen verschiedener Phosphoniumsalze oder Mischungen von Phosphoniumsalzen mit anderen die Iminooxadiazindionbildung beschleunigenden Katalysatoren eingesetzt werden.

Besonders bevorzugte Katalysatoren sind quaternäre Phosphoniumpolyfluoride der Formel R₄P⁺F⁻· n(HF), wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können und n beliebige Werte zwischen 0,1 und 20 annehmen kann.

Es können einzelne Phosphoniumpolyfluoride der Formel R₄P⁺ F^{-.} n(HF), ebenso wie Gemische dieser Salze oder Mischungen von Phosphoniumpolyfluoriden der Formel R₄P⁺ F^{-.} n(HF) mit anderen, die Iminooxadiazindionbildung beschleunigenden Katalysatoren eingesetzt werden.

Ein weiterer Gegenstand der Erfindung ist ein Verfahren zur Herstellung von Iminooxadiazindiongruppen enthaltenden Polyisocyanaten, bei dem
a) mindestens ein organisches Di- und/oder Triisocyanat,
b) ein oder mehrere Katalysatoren, deren Wassergehalte summarisch 1000 ppm nicht überschreiten,
c) optional Lösemittel und
d) optional Additive
zur Reaktion gebracht werden.

Ein weiterer Gegenstand der Erfindung ist ein Verfahren zur Herstellung von Iminooxadiazindiongruppen enthaltenden Polyisocyanaten, bei dem
a) mindestens ein organisches Di- und/oder Triisocyanat,
b) ein oder mehrere Katalysatoren, enthaltend mindestens ein erfindungsgemäß zu verwendendes Phosphoniumsalz, wobei die Wassergehalte der Katalysatoren summarisch 1000 ppm nicht überschreiten,
c) optional Lösemittel und
d) optional Additive
zur Reaktion gebracht werden.

Ein weiterer Gegenstand der Erfindung ist ein Verfahren zur Herstellung von Iminooxadiazindiongruppen enthaltenden Polyisocyanaten, bei dem
a) mindestens ein organisches Di- und/oder Triisocyanat,
b) ein oder mehrere Katalysatoren, enthaltend mindestens ein erfindungsgemäß zu verwendendes quaternäres Phosphoniumpolyfluorid, wobei die Wassergehalte der Katalysatoren summarisch 1000 ppm nicht überschreiten,
c) optional Lösemittel und
d) optional Additive
zur Reaktion gebracht werden.

Als Additive sind hierbei den Wassergehalt des Katalysators per se nicht beeinflussende Stoffe wie Alkohole, Stabilisatoren (z.B. sterisch gehinderte Phenole oder Amine), Antioxidationsmittel etc. zu verstehen, die in der Polyurethanchemie üblicherweise Verwendung finden.

Das erfindungsgemäße Verfahren kann im Temperaturbereich zwischen 0 °C und + 250 °C, bevorzugt 20 bis 180 °C, besonders bevorzugt 40 bis 150 °C durchgeführt werden und bei beliebigen Umsetzungsgraden, bevorzugt nachdem 5 bis 80 %, besonders bevorzugt 10 bis 60 %, des eingesetzten monomeren Diisocyanates umgesetzt wurden, unterbrochen werden.

Der Katalysatorbedarf im erfindungsgemäßen Verfahren unterscheidet sich dabei nicht von dem in der Bulk-Modifizierung des Standes der Technik beobachteten. Der Katalysator kann beispielweise in einem Anteil zwischen 1 mol-ppm und 1 mol-%, bevorzugt zwischen 5 mol-ppm und 0,1 mol-%, bezogen auf die Monomermenge, eingesetzt werden.

Der Katalysator kann im erfindungsgemäßen Verfahren unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. aliphatische oder aromatische Kohlenwasserstoffe, Alkohole, Ketone, Ester sowie Ether. Bevorzugt werden Alkohole verwendet.

Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration, u.s.w..

Im Anschluss an die Katalysatordesaktivierung kann das nicht umgesetzte Monomer sowie gegebenenfalls mitverwendetes Lösemittel mit Hilfe aller bekannten Separationstechniken wie z.B. Destillation, gegebenenfalls in der speziellen Ausführungsform der Dünnschichtdestillation Extraktion oder Kristallisation/Filtration abgetrennt werden. Selbstverständlich können auch Kombinationen zweier oder mehrerer dieser Techniken angewendet werden.

Soll das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordesaktivierung auf die Monomerenabtrennung verzichtet werden.

Bevorzugt wird das nicht umgesetzte Monomer abgetrennt. Bevorzugt weisen die erfindungsgemäßen Produkte nach der Abtrennung einen Restmonomergehalt < 0,5 % bevorzugt < 0,1 Gew.- % auf.

Bevorzugt wird das nicht umgesetzte Monomer destillativ abgetrennt.

Verglichen mit der Katalyse z.B. durch quaternäre Phosphoniumsalze ohne Verwendung von Additiven, die dem Katalysator das Wasser entziehen (Bulk-Modifizierung, s. Vergleichsbeispiel), beobachtet man im erfindungsgemäßen Verfahren bei sonst gleichen Reaktionsbedingungen eine deutlich verbesserte Katalysatorstabilität was sich in deutlich niedrigeren Phosphorgehalten des Recyclates äußert (vgl. erfindungsgemäße Beispiele).

Die im erfindungsgemäßen Verfahren resultierenden monomerenarmen Iminooxadiazindiongruppen aufweisenden Polyisocyanate weisen das gleiche hohe Qualitätsniveau auf, wie die Produkte, die nach vorbeschriebenen Verfahren des Standes der Technik erhalten werden und sind von ihnen analytisch nicht unterscheidbar.

Nach einer besonderen, kontinuierlich arbeitenden Ausführungsform des erfindungsgemäßen Verfahrens kann die Oligomerisierung in einem Rohrreaktor vorgenommen werden. Hierbei profitiert man ebenfalls von der geringeren Zersetzungstendenz der erfindungsgemäßen Katalysatoren.

Zur Durchführung des erfindungsgemäßen Verfahrens können prinzipiell alle bekannten (Di)isocyanate des Standes der Technik einzeln oder in beliebigen Abmischungen untereinander eingesetzt werden.

Insbesondere seien genannt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

Bevorzugt werden eingesetzt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI) sowiel,3- sowie 1,4-Bis(isocyanatomethyl)cyclohexan (H6XDI).

Hierbei ist es belanglos, nach welchen Verfahren die vorstehend genannten (Poly)isocyanate generiert werden, d.h. mit oder ohne Verwendung von Phosgen.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Die nachfolgenden Vergleichsbeispiele und **Beispiele** sollen die Erfindung näher erläutern, ohne sie jedoch einzuschränken.

### Beispiele

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN 53 185.

Der Phosphorgehalt aller Proben wurde durch Röntgenfluoreszenzanalyse (RFA) bestimmt.

Der Wassergehalt der Katalysatorlösungen wurde mittels Karl-Fischer-Titration nach DIN DIN 51777-2 bestimmt.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skale wurden geringe Mengen von Tetramethylsilan im Lösungsmittel mit 0 ppm ¹H-NMR-chem. Verschiebung heran gezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung.. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 896 009.

Die dynamischen Viskositäten wurden bei 23 °C mit dem Viskosimeter VT 550 der Fa. Haake bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen. Die Herstellung der Hydrogenpolyfluoridkatalysatoren wird u.a. in EP-A 962454 und darin zitierte Literatur beschrieben.

### Beispiel 1 Vergleichsbeispiel

In einem doppelwandigen, durch einen externen Kreislauf auf 60°C temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurden 507 mg einer ca. 70%igen isopropanolischen Lösung von Tetrabutylphosphoniumhydrogendifluorid mit 2200 ppm Wassergehalt und 7,6 % Phosphorgehalt portionsweise so dosiert, dass die Temperatur der Reaktionsmischung 65°C nicht überschritt. Nachdem ca. 1 mol NCO Gruppen umgesetzt waren, wurde der Katalysator durch Zugabe einer zum Katalysator äquivalenten Menge an p-Toluolsulfonsäure (als 40%ige Lösung in Isopropanol) deaktiviert, weitere 30 min bei Reaktionstemperatur nachgerührt und anschließend aufgearbeitet. Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120°C, KWV-Temp.: 140°C), wobei nicht umgesetztes Monomer als Destillat und das monomerenarme Polyisocyanatharz als Sumpfprodukt separiert wurden (Startdurchlauf, Beispiel 1-A). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde insgesamt fünfmal wiederholt (Katalysatordosen: 456 mg; 501 mg; 490 mg; 446 mg und 458 mg). Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 92 % Wiederfindung auf 79 % des gefundenen Phosphors in den Harzen und 21 % im letzten Destillat. Die gemittelten Daten der in den Versuchen 1-B bis 1-F erhaltenen Polyisocyanatharze lauten wie folgt:
Harzausbeute (bezogen auf eingesetztes HDI): 17,6%
NCO-Gehalt: 23,4 %
Viskosität: 700 mPas/23°C
Iminooxadiazindione: 51 mol-%*
Isocyanurate: 43 mol-%*
Uretdione: 6 mol-%*
* = bezogen auf die Summe der in der Modifizierungsreaktion gebildeten NCO-Folgeprodukte

### Beispiel 2 erfindungsgemäß

Es wurde Verfahren wie im Vergleichsbeispiel 1 beschrieben, mit dem Unterschied, dass der Wassergehalt des verwendeten Katalysators vorher durch Zugabe einer dem Wassergehalt äquimolaren Menge an Trimethylorthoacetat zur Katalysatorlösung auf 360 ppm abgesenkt worden war.

Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 95 % Wiederfindung auf 88 % des gefundenen Phosphors in den Harzen und 12 % im letzten Destillat. Die gemittelten Daten der in den Versuchen 2-B bis 2-F erhaltenen Polyisocyanatharze lauten wie folgt:
Harzausbeute (bezogen auf eingesetztes HDI): 18,1 %
NCO-Gehalt: 23,5 %
Viskosität: 695 mPas/23°C
Iminooxadiazindione: 53 mol-%*
Isocyanurate: 42 mol-%*
Uretdione: 5 mol-%*
* = bezogen auf die Summe der in der Modifizierungsreaktion gebildeten NCO-Folgeprodukte

### Beispiel 3 erfindungsgemäß

Es wurde Verfahren wie im Vergleichsbeispiel 1 beschrieben, mit dem Unterschied, dass der Wassergehalt des verwendeten Katalysators vorher durch Zugabe einer dem Wassergehalt äquimolaren Menge an Triethylorthoacetat zur Katalysatorlösung auf 410 ppm abgesenkt worden war.

Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 92 % Wiederfindung auf 91 % des gefundenen Phosphors in den Harzen und 9 % im letzten Destillat. Die gemittelten Daten der in den Versuchen 3-B bis 3-F erhaltenen Polyisocyanatharze lauten wie folgt:
Harzausbeute (bezogen auf eingesetztes HDI): 18,4 %
NCO-Gehalt: 23,5 %
Viskosität: 705 mPas/23°C
Iminooxadiazindione: 52 mol-%*
Isocyanurate: 43 mol-%*
Uretdione: 5 mol-%*
* = bezogen auf die Summe der in der Modifizierungsreaktion gebildeten NCO-Folgeprodukte

### Beispiel 4 erfindungsgemäß

Es wurde Verfahren wie im Vergleichsbeispiel 1 beschrieben, mit dem Unterschied, dass der Wassergehalt des verwendeten Katalysators vorher durch Azeotropieren mit Toluol und anschließende destillative Entfernung des Toluols bei sukzessivem Ersatz durch 2-Ethylhexanol auf 580 ppm abgesenkt worden war. Der Phosphorgehalt der Katalysatorlösung lag anschließend bei 7,3 %.

Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 95 % Wiederfindung auf 92 % des gefundenen Phosphors in den Harzen und 8 % im letzten Destillat. Die gemittelten Daten der in den Versuchen 4-B bis 4-F erhaltenen Polyisocyanatharze lauten wie folgt:
Harzausbeute (bezogen auf eingesetztes HDI): 19,1 %
NCO-Gehalt: 23,3 %
Viskosität: 720 mPas/23°C
Iminooxadiazindione: 49 mol-%*
Isocyanurate: 46 mol-%*
Uretdione: 5 mol-%*
* = bezogen auf die Summe der in der Modifizierungsreaktion gebildeten NCO-Folgeprodukte

## Patentansprüche

1. Verwendung von Katalysatoren bei der Herstellung von Iminooxadiazindiongruppen enthaltenden Polyisocyanaten durch Oligomerisierung monomerer Di- und/oder Triisocyanate, **dadurch gekennzeichnet, dass** der Wassergehalt der Katalysatoren bei der Zugabe zu den zu oligomerisierenden Isocyanaten 1000 ppm nicht überschreitet.

2. Verwendung gemäß Anspruch 1, **dadurch gekennzeichnet, dass** die Katalysatoren solche auf Basis von quarternären Phosphoniumsalzen sind, deren Kationen der allgemeinen Formel R₄P⁺ entsprechen, wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können.

3. Verwendung gemäß Anspruch 2, **dadurch gekennzeichnet, dass** die Katalysatoren quaternäre Phosphoniumpolyfluoride der Formel R₄P⁺ F^{-.} n(HF) sind, wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können und n beliebige Werte zwischen 0,1 und 20 annehmen kann.

4. Verfahren zur Herstellung von Iminooxadiazindiongruppen enthaltenden Polyisocyanaten, bei dem
a) mindestens ein organisches Di- und/oder Triisocyanat,
b) ein oder mehrere Katalysatoren, deren Wassergehalte summarisch 1000 ppm nicht überschreiten,
c) optional Lösemittel und
d) optional Additive
zur Reaktion gebracht werden.

5. Verfahren gemäß Anspruch 4, bei dem
a) mindestens ein organisches Di- und/oder Triisocyanat,
b) ein oder mehrere Katalysatoren, enthaltend mindestens ein erfindungsgemäß zu verwendendes Phosphoniumsalz, wobei die Wassergehalte der Katalysatoren summarisch 1000 ppm nicht überschreiten,
c) optional Lösemittel und
d) optional Additive
zur Reaktion gebracht werden.

6. Verfahren gemäß Anspruch 4, bei dem
a) mindestens ein organisches Di- und/oder Triisocyanat,
b) ein oder mehrere Katalysatoren, enthaltend mindestens ein erfindungsgemäß zu verwendendes quaternäres Phosphoniumpolyfluorid, wobei die Wassergehalte der Katalysatoren summarisch 1000 ppm nicht überschreiten,
c) optional Lösemittel und
d) optional Additive
zur Reaktion gebracht werden.

7. Verfahren gemäß einem der Ansprüche 4 bis 6, **dadurch gekennzeichnet, dass** das Verfahren im Temperaturbereich zwischen 0 °C und + 250 °C durchgeführt wird.

8. Verfahren gemäß einem der Ansprüche 4 bis 7, **dadurch gekennzeichnet, dass** das Verfahren nachdem 5 bis 80 % des eingesetzten monomeren Diisocyanates umgesetzt wurden, unterbrochen werden.

9. Verfahren gemäß einem der Ansprüche 4 bis 8, **dadurch gekennzeichnet, dass** der Katalysator in einem Anteil zwischen 1 mol-ppm und 1 mol-%, bezogen auf die Monomermenge, eingesetzt werden.

10. Verfahren gemäß einem der Ansprüche 4 bis 9, **dadurch gekennzeichnet, dass** das nicht umgesetzte Monomer aus der Reaktionsmischung abgetrennt wird.

11. Verfahren gemäß einem der Ansprüche 4 bis 10, **dadurch gekennzeichnet, dass** mindestens ein Isocyanat in das Verfahren eingesetzt wird ausgewählt aus der Gruppe umfassend Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiiso-cyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) und mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

## Claims

1. Use of catalysts for preparing polyisocyanates containing iminooxadiazinedione groups by oligomerization of monomeric di- and/or triisocyanates, **characterized in that** the water content of the catalysts added to the isocyanates to be oligomerized does not exceed 1000 ppm.

2. Use according to Claim 1, **characterized in that** the catalysts are those based on quarternary phosphonium salts having cations corresponding to the general formula R₄P⁺, where R are the same or different, optionally branched, aliphatic, aromatic and/or araliphatic C₁-C₂₀ residues and optionally two or more substituents R may also form saturated or unsaturated rings with one another and with the phosphorus atom.

3. Use according to Claim 2, **characterized in that** the catalysts are quaternary phosphonium polyfluorides of the formula R₄P⁺ F⁻ · n (HF), where R are the same or different, optionally branched, aliphatic, aromatic and/or araliphatic C₁-C₂₀ residues and optionally two or more substituents R may also form saturated or unsaturated rings with one another and with the phosphorus atom and n can have any values between 0.1 and 20.

4. Method for preparing polyisocyanates containing iminooxadiazinedione groups which comprises reacting
a) at least one organic di- and/or triisocyanate,
b) one or more catalysts having water contents not exceeding 1000 ppm in total,
c) optionally solvent and
d) optionally additives.

5. Method according to Claim 4, which comprises reacting
a) at least one organic di- and/or triisocyanate,
b) one or more catalysts, comprising at least one phosphonium salt to be used in accordance with the invention, wherein the water contents of the catalysts do not exceed 1000 ppm in total,
c) optionally solvent and
d) optionally additives.

6. Method according to Claim 4, which comprises reacting
a) at least one organic di- and/or triisocyanate,
b) one or more catalysts, comprising at least one quaternary phosphonium polyfluoride to be used in accordance with the invention, wherein the water contents of the catalysts do not exceed 1000 ppm in total,
c) optionally solvent and
d) optionally additives.

7. Method according to any of Claims 4 to 6, **characterized in that** the method is carried out in the temperature range between 0°C and + 250°C.

8. Method according to any of Claims 4 to 7, **characterized in that** the method is interrupted after 5 to 80% of the monomeric diisocyanate used have reacted.

9. Method according to any of Claims 4 to 8, **characterized in that** the catalyst is used in a proportion between 1 mol ppm and 1 mol%, based on the amount of monomer.

10. Method according to any of Claims 4 to 9, **characterized in that** the unreacted monomer is removed from the reaction mixture.

11. Method according to any of Claims 4 to 10, **characterized in that** at least one isocyanate used in the method is selected from the group comprising hexamethylene diisocyanate (HDI), 2-methylpentane-1,5-diisocyanate, 2,4,4-trimethyl-1,6-hexanediisocyanate, 2,2,4-trimethyl-1,6-hexanediisocyanate, 4-isocyanatomethyl-1,8-octanediisocyanate, 3(4)-isocyanatomethyl-1-methylcyclohexyl isocyanate (IMCI), isophorone diisocyanate (IPDI), 1,3- and 1,4-bis(isocyanatomethyl)benzene (XDI), 1,3- and 1,4-bis(isocyanatomethyl)cyclohexane (H6XDI), 2,4- and 2,6-toluylene diisocyanate (TDI), bis (4-isocyanatophenyl)methane (4,4'MDI), 4-isocyanatophenyl-2-isocyanatophenylmethane (2,4'MDI) and polycyclic products which are accessible by formaldehyde-aniline polycondensation and subsequent conversion of the resulting (poly)amines to the corresponding (poly)isocyanates (polymer-MDI).

## Revendications

1. Utilisation de catalyseurs lors de la préparation de polyisocyanates contenant des groupes iminooxadiazinedione par oligomérisation de diisocyanates et/ou de triisocyanates monomères, **caractérisée en ce que** la teneur en eau des catalyseurs lors de l'addition aux isocyanates à oligomériser ne dépasse pas 1000 ppm.

2. Utilisation selon la revendication 1, **caractérisée en ce que** les catalyseurs sont des catalyseurs à base de sels de phosphonium quaternaire, dont les cations correspondent à la formule générale R₄P⁺, R représentant des radicaux identiques ou différents, le cas échéant ramifiés, aliphatiques, aromatiques et/ou araliphatiques en C₁-C₂₀ et, le cas échéant, deux substituants R ou plus pouvant également former, les uns avec les autres et avec l'atome de phosphore, des cycles saturés ou insaturés.

3. Utilisation selon la revendication 2, **caractérisée en ce que** les catalyseurs sont des polyfluorures de phosphonium de formule R₄P⁺F⁻*n(HF), R représentant des radicaux identiques ou différents, le cas échéant ramifiés, aliphatiques, aromatiques et/ou araliphatiques en C₁-C₂₀ et, le cas échéant, deux substituants R ou plus pouvant également former, les uns avec les autres et avec l'atome de phosphore, des cycles saturés ou insaturés et n pouvant représenter des valeurs quelconques entre 0,1 et 20.

4. Procédé pour la préparation de polyisocyanates contenant des groupes iminooxadiazinedione, dans lequel on fait réagir
a) au moins un diisocyanate et/ou triisocyanate organique,
b) un ou plusieurs catalyseurs, dont la somme des teneurs en eau ne dépasse pas 1000 ppm,
c) éventuellement des solvants et
d) éventuellement des additifs.

5. Procédé selon la revendication 4, dans lequel on fait réagir
a) au moins un diisocyanate et/ou triisocyanate organique,
b) un ou plusieurs catalyseurs, contenant au moins un sel de phosphonium à utiliser selon l'invention, la somme des teneurs en eau des catalyseurs ne dépassant pas 1000 ppm,
c) éventuellement des solvants et
d) éventuellement des additifs.

6. Procédé selon la revendication 4, dans lequel on fait réagir
a) au moins un diisocyanate et/ou triisocyanate organique,
b) un ou plusieurs catalyseurs, contenant au moins un polyfluorure de phosphonium quaternaire à utiliser selon l'invention, la somme des teneurs en eau des catalyseurs ne dépassant pas 1000 ppm,
c) éventuellement des solvants et
d) éventuellement des additifs.

7. Procédé selon l'une quelconque des revendications 4 à 6, **caractérisé en ce que** le procédé est réalisé dans la plage de température entre 0 et +250°C.

8. Procédé selon l'une quelconque des revendications 4 à 7, **caractérisé en ce que** le procédé est interrompu après la transformation de 5 à 80% du diisocyanate monomère utilisé.

9. Procédé selon l'une quelconque des revendications 4 à 8, **caractérisé en ce que** le catalyseur est utilisé en une proportion entre 1 ppm en mole et 1% en mole, par rapport à la quantité de monomères.

10. Procédé selon l'une quelconque des revendications 4 à 9, **caractérisé en ce que** le monomère non transformé est séparé du mélange réactionnel.

11. Procédé selon l'une quelconque des revendications 4 à 10, **caractérisé en ce qu'**on utilise, dans le procédé, au moins un isocyanate choisi dans le groupe comprenant le diisocyanate d'hexaméthylène (HDI), le 1,5-diisocyanate de 2-méthylpentane, le diisocyanate de 2,4,4-triméthyl-1,6-hexane, le diisocyanate de 2,2,4-triméthyl-1,6-hexane, le diisocyanate de 4-isocyanatométhyl-1,8-octane, l'isocyanate de 3(4)-isocyanatométhyl-1-méthylcyclohexyle (IMCI), le diisocyanate d'isophorone (IPDI), le 1,3-bis(isocyanatométhyl)benzène ainsi que le 1,4-bis(isocyanatométhyl)benzène (XDI), le 1,3-bis(isocyanatométhyl)cyclohexane et le 1,4-bis(isocyanatométhyl)cyclohexane (H6XDI), le diisocyanate de 2,4-toluylène et de 2,6-toluylène (TDI), le bis(4-isocyanatophényl)méthane (4,4'MDI), le 4-isocyanatophényl-2-isocyanatophénylméthane (2,4'MDI) et les produits à plusieurs noyaux, qui sont accessibles par polycondensation de formaldéhyde-aniline et transformation consécutive des (poly)amines obtenues en (poly)isocyanates correspondants (MDI polymère).
